Europäisches Patentamt

(19) European Patent Office

Office européen des brevets

(11) **EP 0 777 491 B1**

(12) **EUROPÄISCHE PATENTSCHRIFT**

(45) Veröffentlichungstag und Bekanntmachung des Hinweises auf die Patenterteilung:
**17.11.2004 Patentblatt 2004/47**

(51) Int Cl.⁷: **A61K 38/01**, A61K 38/23
// (A61K38/01, 31:57),
(A61K38/01, 33:06),
(A61K38/23, 38:01, A61P19:10)

(21) Anmeldenummer: **95929893.6**

(22) Anmeldetag: **19.08.1995**

(86) Internationale Anmeldenummer:
**PCT/EP1995/003304**

(87) Internationale Veröffentlichungsnummer:
**WO 1996/005851 (29.02.1996 Gazette 1996/10)**

(54) **VERWENDUNG VON GESCHMACKSNEUTRALEM, HYDROLYSIERTEM KOLLAGEN UND MITTEL ENTHALTEND DASSELBE**

USE OF TASTELESS, HYDROLYSED COLLAGEN AND AGENT CONTAINING THE SAME

UTILISATION DE COLLAGENE HYDROLYSE, SANS GOUT, ET SUBSTANCE CONTENANT LEDIT COLLAGENE

(84) Benannte Vertragsstaaten:
**AT BE CH DE DK ES FR GB GR IE IT LI LU MC NL PT SE**

(30) Priorität: **23.08.1994 DE 4429842**

(43) Veröffentlichungstag der Anmeldung:
**11.06.1997 Patentblatt 1997/24**

(73) Patentinhaber: **Deutsche Gelatine-Fabriken Stoess AG**
**69402 Eberbach (DE)**

(72) Erfinder:
• **MILAN, Adam**
**130 00 Prag 3 (CZ)**
• **EGGERSGLÜSS, Bernd**
**D-69434 Heddesbach (DE)**
• **BRÄUMER, Klaus**
**D-69412 Eberbach (DE)**
• **SCHRIEBER, Reinhard**
**D-69412 Eberbach (DE)**

(74) Vertreter:
**Werner, Hans-Karsten, Dr.Dipl.-Chem. et al**
**Patentanwälte**
**Von Kreisler-Selting-Werner**
**Postfach 10 22 41**
**50462 Köln (DE)**

(56) Entgegenhaltungen:
**EP-A- 0 254 289          CH-A- 494 250**
**CN-A- 1 077 630**

• **ERFAHRUNGSHEILKUNDE, Bd. 28, Nr. 11, November 1979 HEIDELBERG, DE, Seiten 930-938, E. KRUG 'ZUR UNTERSTÜTZENDEN THERAPIE BEI OSTEO- UND CHONDROPATHIEN.'**

Bemerkungen:
Die Akte enthält technische Angaben, die nach dem Eingang der Anmeldung eingereicht wurden und die nicht in dieser Patentschrift enthalten sind.

## Beschreibung

Gegenstand der Erfindung sind die Verwendung von geschmacksneutralem, enzymatisch hydrolysiertem Kollagen aus tierischer Haut mit einem mittleren Molekulargewicht von 1 bis 40 kD sowie Mittel enthaltend dasselbe.

Aus der EP-B-0 254 289 sind bekannt Mittel zur Behandlung von Arthrosen enthaltend geschmacksloses oder geschmacksneutrales, enzymatisch hydrolysiertes Kollagen aus tierischer Haut, tierischen Knochen, ausreichend gereinigtem Bindegewebe oder Gelatine mit einem mittleren Molekulargewicht von 10 bis 80 kD.

Die DE-A-36 26 414 beschreibt ein Präparat zur Stimulierung von Chondrocyten und Osteoblasten (Ossein-Hydroxyapatit-Komplex) sowie Verfahren zu seiner Herstellung und dieses enthaltende Arzneimittel. Das Präparat wird gewonnen durch Reinigen und Mahlen von Knochen und enthält daher natives Kollagen. Wichtigster Bestandteil des Präparates ist jedoch der Ossein-Hydroxyapatit-Komplex mit seinem hohen Gehalt an Calcium und Phosphat.

Die JP-A-05 05 1400 beschreibt die Herstellung eines Präparates aus Zellkulturen von Kaninchenknorpel. Das Kollagen besitzt ein Molekulargewicht von 60 kD und besteht aus dem Typ II.

Die GB-PS-1,227,534 beschreibt die Herstellung eines Kollagenhydrolysats durch alkalische oder saure Druckhydrolyse. Dieses Präparat enthält außer degradiertem Kollagen 4 bis 15 % freie Aminosäuren. Das Ausgangsmaterial wird nicht näher spezifiziert. Dieses Präparat soll vorzugsweise zusammen mit Calciumsalzen zum Einsatz kommen und die Zufuhr erhöhter Calciummengen ermöglichen.

Osteoporose ist definiert als die klinische Manifestation von Knochenschwund. Dies bedeutet eine Reduktion der Knochenmasse bezogen auf das Volumen. Zur Zeit sind über 20 Ursachen für das Auftreten von Osteoporose bekannt. Die am häufigsten auftretenden Formen sind die postmenopausale und senile Osteoporose. Mit Beginn des fünften Lebensjahrzehnts beginnt die gesamte Knochenmasse sich zu verringern.

Ursache dafür ist die Tatsache, daß dann mehr Knochenmasse abgebaut als aufgebaut wird. Osteoporose ist die am weitest verbreitete Stoffwechselerkrankung des Knochens, welche mit dem Alter zunimmt, und zwar überwiegend bei Frauen.

Drei bis fünf Jahre nach der Menopause tritt gewöhnlich ein plötzlicher Verlust an Knochenmasse auf. Dies manifestiert sich im Auftreten von Frakturen der Wirbelsäule, Brüche des Oberschenkelhalses und des Unterarmes. Zum Zeitpunkt des ersten Auftretens einer Fraktur sind meist nur noch ca. 50 % der Knochenmasse erhalten geblieben.

Die Frühdiagnose von Osteoporose stellt ein großes Problem dar, da man im normalen Röntgenbild erst nach einem Verlust von mindestens 30 % der Knochenmasse etwas erkennen kann.

Bei osteoporotischen Veränderungen wird auch die Menge der Kollagenfasern des Knochens reduziert. Die Abbauprodukte dieses Kollagen-Katabolismus können vermehrt im Urin nachgewiesen werden.

Vom molekularen Standpunkt gesehen, hat der Knochen eine einmalige Kollagenzusammensetzung. Er ist das einzige Gewebe, in. welchem Kollagen Typ I nicht von Kollagen Typ III begleitet wird. Die Kollagenstrukturen werden durch Quervernetzungen stabilisiert, die über zwei Aminosäurereste von Lysin und Hydroxylysin erfolgen.

Es sind noch mehrere andere Quervernetzungsmöglichkeiten beschrieben worden. Fujimoto (Biochem. Biophys. Res. Commun. 1977; 76: 1124 - 1129) beschreibt Quervernetzungselemente, die aus 3 Lysin- bzw. Hydroxylysinresten entstanden sind, es sind dies Pyridinolin (PD) und Desoxypyridinolin (DPD).

Ihr Vorkommen ist bindegewebsspezifisch, aber weder auf einen Gewebetyp noch auf einen Kollagentyp beschränkt. Bekannt ist, daß Kollagene des Typs I, II, III, IX, X und XI diese Art der Quervernetzungen ausbilden können.

Diese Quervernetzungsverbindungen werden vom Körper nicht abgebaut und mit dem Urin ausgeschieden. Der Gehalt im Urin ist deshalb ein guter Indikator für den Kollagenabbau.

Mittel zur Behandlung der Osteoporose sind unter anderem Calcitonin, Calciumsalze wie Calciumfluorid und Calciumbisphosphonate sowie Progesteron.

Die vorliegende Erfindung hat sich die Aufgabe gestellt, eine weitere Verwendung für das aus der EP 0 254 289 bekannte Mittel zur Verfügung zu stellen. Diese Aufgabe konnte jetzt gelöst werden durch die Verwendung von geschmacksneutralem enzymatisch hydrolysiertem Kollagen aus tierischer Haut mit einem mittleren Molekulargewicht von 1 bis 40 kD zur Herstellung von Mitteln zur Behandlung der postmenopausalen Osteoporose. Das Kollagen stammt aus Haut und weist daher als Hauptkomponente die Typen I und III auf. Gelatine selbst besitzt meist ein mittleres Molekulargewicht von 100 bis 500 kD. Die Molekulargewichte werden bestimmt durch HPLC mit nichtglobulären Eichpeptiden aus Kollagen. Diese werden meist gewonnen durch Bromcyan-Abbau. Die Werte für die Molekulargewichte in der EP-B-0 254 289 wurden noch mit globulären Eichproteinen bestimmt und liegen daher deutlich höher als heute mit HPLC und nichtglobulären Eichproteinen gemessen wird.

Die erfindungsgemäß hergestellten Mittel werden vorzugsweise zubereitet als Pasten, Sirupe, Lösungen, Granulate, Komprimate oder instantisierte Pulver. Diesen Mitteln können zusätzlich zugegeben werden Geschmacksstoffe, Süßungsmittel, Mineralstoffe, Vitamine und/oder Aromastoffe.

Vorzugsweise werden sie abgepackt in Dosisform, welche 0,5 bis 12 g hydrolysiertes Kollagen enthält.

Schließlich wurde festgestellt, daß diese Mittel besonders wirksam sind, wenn ihnen zusätzlich die bekannten Mittel zur Behandlung der Osteoporose zugegeben werden wie Calcitonin, die oben genannten Calciumsalze und/ oder Progesteron.

Gegenstand der vorliegenden Erfindung ist somit obendrein das Mittel zur Behandlung der postmenopausalen Osteoporose enthaltend geschmacksneutrales, enzymatisch hydrolysiertes Kollagen aus tierischer Haut mit einem mittleren Molekulargewicht von 1 bis 40 kD und zusätzlich Calcitonin, und/oder Progesteron.

Enzymatisch Hydrolysiertes Kollagen kann nach den üblichen bekannten Verfahren gewonnen werden. Gelatine nach dem Verfahren, wie sie beschrieben wird in "The Science and Technology of Gelatine". A.G. Ward and A. Courts Academic Press 1977. Die Herstellung niedrigmolekularer, kaltwasserlöslicher Kollagenhydrolysate ist beschrieben in: "Enzymatic Hydrolysis of Food Proteins" Jens Adler-Nielsen, Elsevier Applied Science Publishers, London and New York. Vorzugsweise wird enzymatisch hydrolisiertes Kollagen aus Haut eingesetzt.

In einer klinischen Studie mit einem bevorzugten Präparat (Gelitasol®, MG 3,5 kD), welches aus tierische Haut gewannen wird, konnte jetzt festgestellt werden, daß enzymatisch hydrolysiertes Kollagen auch gegen postmenopausalen Osteoporose wirksam ist. Das Ziel dieser Studie war, den Effekt festzustellen, den kollagenhydrolysatreiche Ernährung auf den Metabolismus des Knochens ausübt, und zwar ob eine kollagenhydrolysatreiche Diät zusammen mit Calcitonin einen höheren Knochen-Kollagen-Metabolismus bewirkt als alleinige Calcitoninverabreichung.

Die Ergebnisse dieser Behandlung wurden beurteilt an Hand der klinischen Befunde, Röntgenaufnahmen, Osteometrie und chemischen Untersuchungen inklusive der Bestimmung von Pyridinolin und Desoxypyridinolin im Urin.

Es wurde dafür folgende Doppelblindstudie durchgeführt:

Probanden waren Frauen über 40 Jahre mit postmenopausaler Osteoporose, bei denen nach dem Röntgenbefund und densitometrischen Bestimmungen die Knochenmasse weniger als 80 % betrug.

Folgende Exklusionskriterien kamen zur Anwendung:

- schwere Gelenk- und Bandscheibenleiden unabhängig davon, ob sie entzündlichen, stoffwechselbedingten oder degenerativen Ursprungs waren
- chronische systemische Infektionskrankheiten
- Funktionstörungen von Niere oder Leber
- intensive Osteoporosetherapie
- vorausgegangene (1 Jahr vor Studienbeginn) oder bestehende corticosteriodale Therapie
- bösartige Erkrankungen.

## Patientinnen

121 Frauen nach der Menopause, die laut Röntgendiagnose eindeutig an Osteoporose litten, wurden für diese Studie ausgewählt.

27 dieser Patientinnen beendeten die Studie vorzeitig + hauptsächlich wegen Calcitonin-Unverträglichkeit (Übelkeit, Erbrechen, Hitzewallungen).

## Therapie

Die verbliebenen 94 Patientinnen wurden in zwei randomisierte Gruppen eingeteilt, wobei zufälligerweise in jeder Gruppe je 47 für die gesamte Doppelblind-Studie übrigblieben.

Die eine Gruppe erhielt eine kollagenhydrolysatreiche Diät und wurde zusätzlich mit Calcitonin behandelt. Die andere Gruppe wurde nur mit Calcitonin behandelt und als Placebo Laktose. Die Calcitoninbehandlung wurde zweimal pro Woche durchgeführt, und zwar 100 U i.m. Die Untersuchung wurde für einen Zeitraum von 24 Wochen durchgeführt.

Die anthropometrischen und spezifischen Daten der Patientinnen sind aus Tabelle 1 und die Risikofaktoren aus Tabelle 2 zu ersehen.

Tabelle 1:

| Basisdaten der Probandinnen | | | |
|---|---|---|---|
| | Calcitonin + Kollagenhydrolysat | Calcitonin | Gesamt |
| Patientinnen | 47 | 47 | 94 |
| Alter $\overline{X}$ | 61,43 | 58,94 | 60,18 |
| S | 8,18 | 8,68 | 8,53 |

Tabelle 1:   (fortgesetzt)

| Basisdaten der Probandinnen | | | | |
|---|---|---|---|---|
| | | Calcitonin + Kollagenhydrolysat | Calcitonin | Gesamt |
| Größe        $\overline{X}$ | | 163,09 | 163,11 | 163,10 |
| S | | 5,41 | 7,11 | 6,32 |
| Gewicht        $\overline{X}$ | | 66,17 | 70,64 | 68,40 |
| S | | 10,90 | 15,15 | 13,38 |
| Schwangerschaften | | | 1,94 | 1,93 |
| $\overline{X}$ | | 1,91 | 1,10 | 1,12 |
| S | | 1,15 | | |
| Menses von        $\overline{X}$ | | 13,55 | 13,54 | 13,54 |
| S | | 1,38 | 1,65 | 1,53 |
| $\overline{X}$ | | 48,74 | 47,17 | 47,96 |
| bis        S | | 4,87 | 5,81 | 5,42 |

Tabelle 2:

| Häufigkeit der Risikofaktoren in der entsprechenden Gruppe | | | |
|---|---|---|---|
| | Calcitonin + Kollagenhydrolysat | Calcitonin | Gesamt |
| Patientinnen | 47 | 47 | 94 |
| Bewegungseinschränkung | 30 | 29 | 59 |
| Alkoholabusus | 1 | 1 | 2 |
| Nikotinabusus | 10 | 9 | 19 |
| Coffeinabusus | 7 | 5 | 12 |
| Chronisches Nierenleiden | 0 | 0 | 0 |
| Gastrointestinale Beschwerden | 0 | 0 | 0 |
| Hyperthyreose | 3 | 8 | 11 |
| Hyperparathyreose | 0 | 0 | 0 |
| Corticosteroide | 1 | 0 | 1 |
| Urolithiasis | 2 | 2 | 4 |
| Menstruationsbeschwerden | 7 | 11 | 18 |
| Leistungssport | 7 | 6 | 11 |
| Freizeitsport | 25 | 16 | 41 |

<u>Untersuchungen</u>

Folgende Untersuchungen wurden sowohl vor Beginn als auch nach Beendigung der Studie durchgeführt und bei 61 Patientinnen drei Monate nach Beendigung der Therapie:

- Knochendichte am rechten Unterarm durch single photon absorptiometry-Messung (bei den meisten Patientinnen)
- Röntgenuntersuchung des rechten Unterarms, der Lendenwirbel oder anderer schmerzhafter Wirbelsäulen-Partien
- Serumanalysen
- Pyridinolin- und Desoxypyridinolinbestimmung im Urin
- Hydroxiprolinbestimmung im Urin.

Ergebnisse

Die Abschlußbewertung der therapeutischen Wirksamkeit wurde in einer 3-stufigen Skala bezüglich des subjektiven Befindens der Patientinnen durchgeführt. Die erhaltenen Meßwerte wurden mit STATGRAPHICS-Software (STST Inc. Rockville, MD, USA) statistisch ausgewertet. Der Einfluß von nicht linearen Variablen wurde mittels des einseitigen Varianztests berechnet. Signifikante Unterschiede wurden durch den last significance intervals range test überprüft.

Im Röntgenbild und bei den densitometrischen Messungen konnten keine Unterschiede gefunden werden, welches in Übereinstimmung mit der Veröffentlichung von Villareal et al. (Osteoporosis Int. 1992; 2: 70 - 73) steht. Die Laborwerte - mit Ausnahme von UPD/Kreatinin und U Hydroxiprolin/Kreatinin - waren zu Beginn der Studie normal und blieben während der Behandlung unverändert, siehe Tabelle 3.

Tabelle 3:

| Urinwerte | | | | | | | |
|---|---|---|---|---|---|---|---|
| | | Calcitonin + Kollagenhydrolysat | | Calcitonin | | Gesamt | |
| Monate | | 0 | 6 | 0 | 6 | 0 | 6 |
| Patientinnen | | 47 | | 47 | | 94 | |
| BSG, 1 h | $\bar{X}$ | 18,6 | 15,3 | 18,7 | 17,4 | 18,7 | 18,7 |
| Serum Calcium (mmol/l) | $\bar{X}$ | 2,37 | 2,35 | 2,38 | 2,33 | 2,38 | 2,34 |
| | S | 0,39 | 0,24 | 0,31 | 0,29 | 0,36 | 0,27 |
| Phosphor im Serum (mmol/l) | $\bar{X}$ | 1,18 | 1,29 | 1,17 | 1,25 | 1,18 | 1,27 |
| | S | 0,24 | 0,35 | 0,18 | 0,35 | 0,21 | 0,35 |
| S Alkal. Phosphatase (ukat/l) | $\bar{X}$ | 1,42 | 1,40 | 1,25 | 1,32 | 1,33 | 1,37 |
| | S | 1,24 | 0,69 | 0,45 | 0,65 | 0,93 | 0,68 |
| S Saure Phosphatase (ukat/l) | $\bar{X}$ | 0,21 | 0,18 | 0,18 | 0,18 | 0,19 | 0,18 |
| | S | 0,13 | 0,17 | 0,05 | 0,21 | 0,10 | 0,19 |
| Harncalcium (mmol/mmol creat) | $\bar{X}$ | 0,38 | 0,51 | 0,51 | 0,55 | 0,44 | 0,53 |
| | S | 0,35 | 0,39 | 0,95 | 0,92 | 0,72 | 0,70 |
| Harnphosphat (mmol/mmol creat) | $\bar{X}$ | 2,47 | 2,40 | 2,97 | 2,75 | 2,72 | 2,57 |
| | S | 1,85 | 1,79 | 2,71 | 2,78 | 2,34 | 2,32 |

Die UPD/Kreatinin- und UDPD/Kreatinin-Ausscheidungen fielen von einem anfänglichen Durchschnittswert von 109,6 auf 61,67 nmol/mmol. Es besteht jedoch ein bedeutender Unterschied zwischen den beiden Untersuchungsgruppen. Bei der Gruppe, die mit Kollagenhydrolysat und Calcitonin behandelt wurde, waren die Ursprungswerte von UPD/Kreatinin und UDPD/Kreatinin 114,98 bzw. 23,51 nmol/mmol, und diese verringerten sich nach der Studie auf 58,62 bzw. 11,60 nmol/mmol.

In der Gruppe ohne Kollagenhydrolysat verringerten sich diese Werte um ein viel geringeres Ausmaß, nämlich UPD/Kreatinin von 104,14 auf 22,22 nmol/mmol und UDPD/Kreatinin von 64,73 auf 16,73 nmol/mmol, siehe Tabelle 4.

Im Vergleich mit gesunden Erwachsenen war die Ausscheidung der Kollagenabbauprodukte in beiden Gruppen signifikant erhöht, siehe Tabelle 5.

Tabelle 4:

| Urinwerte der Kollagenabbauprodukte | | | | | | | |
|---|---|---|---|---|---|---|---|
| | | Calcitonin + Kollagenhydrolysat | | Calcitonin | | Gesamt | |
| Monate | | 0 | 6 | 0 | 6 | 0 | 6 |
| Patientinnen | | 47 | | 47 | | 94 | |
| Hydroxyprolin (µmol/mmol Kreatinin) | $\bar{X}$ | 18,8 | 18,9 | 18,0 | 18,1 | 18,4 | 18,5 |
| | S | 10,2 | 13,9 | 12,3 | 9,5 | 11,3 | 12,0 |
| Pyridinolin | $\bar{X}$ | 115,0 | 58,6 | 104,1 | 64,7 | 109,6 | 61,7 |

Tabelle 4:   (fortgesetzt)

| Urinwerte der Kollagenabbauprodukte | | | | | | | |
| --- | --- | --- | --- | --- | --- | --- | --- |
| | | Calcitonin + Kollagenhydrolysat | | Calcitonin | | Gesamt | |
| (nmol/mmol Kreatinin) | S | 65,4 | 21,3 | 37,9 | 21,6 | 53,7 | 21,6 |
| Desoxypyridinolin | $\bar{X}$ | 23,6 | 11,6 | 22,2 | 16,7 | 22,8 | 14,2 |
| (nmol/mmol Kreatinin) | S | 10,4 | 4,9 | 11,4 | 27,9 | 10,9 | 20,2 |
| Knochendichte | $\bar{X}$ | 76,1 | 79,5 | 77,0 | 79,5 | 76,6 | 79,5 |
| (%) | S | 10,0 | 13,9 | 11,2 | 14,3 | 10,7 | 14,1 |

Tabelle 5:

| Urinwerte der Kollagenabbauprodukte - Kontrollgruppe | | |
| --- | --- | --- |
| | | Kontrollgruppe |
| Hydroxyprolin | $\bar{X}$ | 15,7 |
| (µmol/mmol Kreatinin) | S | 8,2 |
| Pyridinolin | $\bar{X}$ | 41,6 |
| (nmol/mmol Kreatinin) | S | 10,6 |
| Desoxypyridinolin | $\bar{X}$ | 8,1 |
| (nmol/mmol Kreatinin) | S | 2,8 |

Die relativen Veränderungen von UPD/Kreatinin, UDPD/Kreatinin und Knochendichte (KnD) sind im Diagramm 1 wiedergegeben.

Diagramm 1

**Pyridinolin** [nmol/mmol Kreat]

**Deoxypyridinolin** [nmol/mmol Kreat]

**KnD** ( % )

1 - Werte vor der Behandlung
2 - Werte nach der Behandlung

Diagramm 1: Der Einfluß der Kollagenhydrolysat - Calcitonintherapie auf die Urinausscheidung von Pyridinolin, Desoxypyridinolin und die Knochenmassendichte.

Das Diagramm 2 zeigt die statistische Signifikanz der Abnahme der beiden biochemischen Marker bei osteoporotischen Patienten im Vergleich zu den Ausgangswerten. Die Unterschiede sind signifikant (5 % level). Andererseits konnte keine Erhöhung der Knochenmassendichte festgestellt werden.

Diagramm 2

Pyridinolin
[nmol/mmol    Kreat]

Deoxypyridinolin
[nmol/mmol Kreat]

KnD
[ % ]

1 - Werte vor der Behandlung
2 - Werte nach der Behandlung

Diagramm 2: Statistische Signifikanz (ca. 95 %) zwischen den Werten vor und nach der Studie.

Die Diagramme 3 und 4 zeigen, daß die Therapie mit Kollagenhydrolysat ein besseres Ergebnis bringt als mit Calcitonin allein.

Diagramm 3

Diagramm 4

95% Vertrauensgrenze

1 - Kollagenhydrolysat + Calcitonin
2 - Calcitonin

Bei Patienten, die drei Monate nach Ende der Therapie untersucht wurden, waren die Pyridinolin- und Desoxypyridinolin-Ausscheidungen im Vergleich zu den Ausgangswerten immer noch reduziert. In der Gruppe, die mit Kollagenhydrolysat behandelt war, war dies ausgeprägter als in der Gruppe, die nur Calcitonin erhalten hatte, siehe Tabelle 6.

Die Veränderungen der Pyridinolin/Kreatinin-Werte bei den mit Kollagenhydrolysat behandelten Patientinnen betrugen nach der Therapie 53,05 %, und drei Monate später sanken sie auf 49,27 %.

Bei mit Calcitonin allein behandelten fielen die entsprechenden Werte von 67,95 auf 67,51 %.

Die Bewertung der subjektiven Aussagen der Patientinnen über die Wirksamkeit der Therapie ergaben, daß die mit Kollagenhydrolysat behandelten eine größere Verbesserung ihres Zustandes testierten als die mit Calcitonin allein behandelten. Es ist bemerkenswert, daß die Verbesserung der subjektiven Symptome mit dem Sinken der Pyridinolin/Kreatinin- und Desoxypyridinolin/Kreatinin-Werte korreliert (siehe Diagramm 5 und 6).

Tabelle 6:

| | Calcitonin + Kollagenhydrolysat | | | Calcitonin | | | Gesamt | | |
|---|---|---|---|---|---|---|---|---|---|
| Monate | 0 | 6 | 9 | 0 | 6 | 9 | 0 | 6 | 9 |
| Patientinnen | 30 | | | 30 | | | 60 | | |
| Hydroxyprolin (μmol/mmol Kreatinin) | 17,7 9,6 | 21,1 16,0 | 12,8 6.1 | 18,1 14,4 | 17,1 8,0 | 14,0 6,2 | 17,9 12,2 | 19,1 12,7 | 13,4 6,2 |
| Pyridinolin (nmol/mmol Kreatinin) | 125,4 76,8 | 59,9 20,9 | 57,0 18,7 | 108,9 39,9 | 69,2 23,1 | 66,2 26,4 | 117,0 61,5 | 64,9 22,5 | 61,7 23,4 |
| Desoxypyridin- | 24,7 | 11,6 | 11,9 | 24,8 | 19,2 | 14,4 | 24,7 | 15,4 | 13,6 |

Tabelle 6:  (fortgesetzt)

| | Calcitonin + Kollagenhydrolysat | | | Calcitonin | | | Gesamt | | |
|---|---|---|---|---|---|---|---|---|---|
| olin (μmol/mmol Kreatinin) | 11,1 | 5,1 | 4,9 | 12,6 | 34,0 | 6,7 | 11,9 | 24,8 | 6,0 |
| (%) Knochendichte | 77,3 8,2 | 81,2 12,1 | 80,7 11,2 | 79,3 9,9 | 82,3 13,4 | 81,9 14,3 | 78,3 9,2 | 81,8 12,8 | 81,3 12,8 |

Diagramm 5

95% Vertrauensgrenze

Subjektives Empfinden:  1 - gering
                        2 - mäßig
                        3 - gut

Relatives Absinken der Anfangswerte der Pyridinolin-Ausscheidung im Urin nach der Therapie im Vergleich mit der subjektiven Beurteilung.

Diagramm 6

95% Vertrauensgrenze

Subjektives Empfinden: 1 - gering
2 - mäßig
3 - gut

Relatives Absinken der Anfangswerte der Desoxypyridinolin-Ausscheidung im Urin nach der Therapie im Vergleich mit der subjektiven Beurteilung.

Schlußfolgerungen:

Das Kollagen des Knochens besteht ausschließlich aus Typ I, und dieses enthält einen ungewöhnlich hohen Anteil an Pyridinolin in Form von Quervernetzungen, mehr als in anderen Bindegeweben vorkommt. Da der Knochen als einziges Gewebe seine Strukturelemente in hohem Maße erneuert (Kamel et al., J. chromatogr. 1992; 574: 255 - 260) wird angenommen, daß das Pyridinolin, welches mit dem Urin ausgeschieden wird, hauptsächlich vom Knochen stammt. Diese Vermutung wird gestützt von dem molaren Verhältnis von Pyridinolin zu Desoxypyridinolin im Urin, welches normalerweise dem des Knochens entspricht.

Das Hydroxyprolin-Kreatinin Verhältnis im Urin erwies sich als nicht geeignet zur Erfassung des Knochenmetabolismus, da dieses zu stark vom Abbau des Kollagens anderer Gewebe überlagert wird sowie ebenso durch das eingenommene Kollagenhydrolysat.

Es ist bekannt, daß die Behandlung von Osteoporosepatienten mit Calcitonin den weiteren Knochenverlust vermindert, es gibt aber bisher keinen Hinweis darauf, daß eine Calcitonintherapie die Knochendichte außer bei Langzeitbehandlung erhöht. Calcitonin hat weiterhin einen analgetischen Effekt, der aber getrennt von der Wirkung auf den Knochen gesehen werden muß. Es ist bekannt, daß nur eine Langzeitbehandlung mit Calcitonin die Knochendichte erhöht. Andererseits stimuliert Gelatine die Zellproliferation in vitro, und es wurde eine positive Wirkung einer kollagenhydrolysatreichen Diät bei Patienten mit Osteoarthrose beobachtet. Darüber hinaus wurde bei diesen Patienten eine verbesserte Wundheilung, ein besseres allgemeines Wohlbefinden und eine Verminderung der Rückenschmerzen festgestellt. Als Ergebnis der Untersuchungen kann festgestellt werden, daß die kollagen-hydrolysatreiche Therapie bei postmenopausalen Osteoporose wohl auf metabolische Einflüsse zurückzuführen ist. Weiterhin ist sicher, daß die Therapie mit Kollagenhydrolysat und Calcitonin stärkere positive Effekte auf den Knochenmetabolismus zeigt als die Behandlung mit Calcitonin allein. Dies war auch noch drei Monate nach den Therapien zu beobachten.

**Patentansprüche**

1.  Verwendung von geschmacksneutralem, enzymatisch hydrolysiertem Kollagen aus tierischer Haut mit einem mittleren Molekulargewicht von 1 bis 40 kD zur Herstellung von Mitteln zur Behandlung von postmenopausalen Osteoporose.

2.  Verwendung gemäß Anspruch 1, **dadurch gekennzeichnet, dass** die Mittel zubereitet werden zu Pasten, Sirupen, Lösungen, Granulaten, Komprimaten oder instantisierten Pulvern.

3.  Verwendung gemäß einem der Ansprüche 1 oder 2, **dadurch gekennzeichnet, dass** den Mitteln zusätzlich Geschmackstoffe, Süßungsmittel, Mineralstoffe, Vitamine und/oder Aromastoffe zugegeben werden.

4.  Verwendung gemäß einem der Ansprüche 1 bis 3, **dadurch gekennzeichnet, dass** die Mittel in Dosisform abgepackt werden, welche 0,5 bis 12 g hydrolysiertes Kollagen enthalten.

5.  Verwendung gemäß einem der Ansprüche 1 bis 4, **dadurch gekennzeichnet, dass** die Mittel zusätzlich enthalten Calcitonin und/oder Progesteron.

6.  Mittel zur Behandlung der postmenopausalen Osteoporose enthaltend geschmacksneutrales, enzymatisch hydrolysiertes Kollagen aus tierischer Haut mit einem mittleren Molekulargewicht von 1 bis 40 kD und zusätzlich Calcitonin und/oder Progesteron.

**Claims**

1.  Use of tasteless, enzymatically hydrolyzed collagen from animal skin having an average molecular weight of from 1 to 40 kD for the preparation of agents for the treatment of postmenopausal osteoporosis.

2.  The use according to claim 1, **characterized in that** said agents are formulated into pastes, syrups, solutions, granules, compressed formulations or instantized powders.

3.  The use according to any of claims 1 or 2, **characterized in that** flavoring agents, sweeteners, minerals, vitamins and/or flavoring agents are additionally added to said agents.

4.  The use according to any of claims 1 to 3, **characterized in that** said agents are packaged in dosage forms containing from 0.5 to 12 g of hydrolyzed collagen.

5.  The use according to any of claims 1 to 4, **characterized in that** said agents additionally contain calcitonin and/or progesterone.

6.  Agents for the treatment of postmenopausal osteoporosis containing tasteless, enzymatically hydrolyzed collagen from animal skin having an average molecular weight of from 1 to 40 kD, and in addition calcitonin and/or progesterone.

**Revendications**

1.  Utilisation de collagène de derme animal hydrolysé enzymatiquement, sans goût défini, d'un poids moléculaire moyen de 1 à 40 kD pour préparer des agents destinés au traitement de l'ostéoporose post-ménopausique.

2.  Utilisation selon la revendication 1, **caractérisée en ce que** les agents sont préparés sous forme de pâtes, sirops, solutés, granulés, comprimés ou poudres à dissolution instantanée.

3.  Utilisation selon une des revendications 1 ou 2, **caractérisée en ce qu'**en complément, on ajoute aux agents des arômes, des édulcorants, des minéraux, des vitamines et/ou des substances aromatiques.

4. Utilisation selon une des revendications de 1 à 3, **caractérisée en ce que** les agents sont conditionnés sous forme de doses, qui contiennent de 0,5 à 12 g de collagène hydrolysé.

5. Utilisation selon une des revendications de 1 à 4, **caractérisée en ce que** les agents contiennent en outre de la calcitonine et/ou de la progestérone.

6. Produit destiné au traitement de l'ostéoporose post-ménopausique contenant du collagène de derme animal, hydrolysé enzymatiquement, sans goût défini, d'un poids moléculaire moyen de 1 à 40 kD et, en outre, de la calcitonine et/ou de la progestérone.